# EUROPEAN PATENT SPECIFICATION

(11) **EP 2 374 450 B1**
(45) Date of publication and mention of the grant of the patent: **27.06.2012**
(21) Application number: 10159137.8
(22) Date of filing: 06.04.2010
(51) Int. Cl.: A61K 31/724, A61K 31/135, A61K 9/20, A61K 31/496, A61K 45/06, A61K 47/40

(54) **Flupentixol compositions**
Flupentixolzusammensetzungen
Compositions de flupentixol

(43) Date of publication of application: 12.10.2011
(73) Proprietor: H. Lundbeck A/S, 2500 Valby (DK)
(72) Inventor: Olsen, Flemming, Enok, 3450, Allerød (DK)

(56) References cited:
- US-A- 5 904 929
- Anonymous: "Deanxit -Dragees"[Online] March 2008 (2008-03), XP002596510 Pharmazie.com Retrieved from the Internet: URL:http://www.pharmazie.com/graphic/A/93/ 0-14693.pdf> [retrieved on 2010-08-12]
- DATABASE WPI Week 200872 Thomson Scientific, London, GB; AN 2008-M13873 XP002596184 & CN 101 125 131 A (SHANGHAI CIRUI MEDICAL SCI CO LTD) 20 February 2008 (2008-02-20)
- DATABASE MEDLINE [Online] US NATIONAL LIBRARY OF MEDICINE (NLM), BETHESDA, MD, US; February 1979 (1979-02), ENEVER R P ET AL: "Flupenthixol dihydrochloride decomposition in aqueous solution." XP002595737 Database accession no. NLM423084 & JOURNAL OF PHARMACEUTICAL SCIENCES FEB 1979 LNKD- PUBMED:423084, vol. 68, no. 2, February 1979 (1979-02), pages 169-171, ISSN: 0022-3549

## Description

### Field of invention

Flupentixol is an antipsychotic of the thioxanthene group. Flupentixol has the structure depicted below 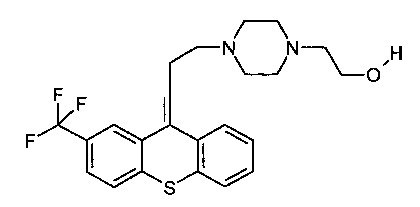 and the systematic name 2-(4-{3-[2-trifluoromethyl-thioxanthen-(9E)-ylidene]-propyl}-piperazin-1-yl)-ethanol. The compound was first disclosed in the GB patent 925538 to Smith Kline & French Laboratories in 1963 and said to have e.g. tranquilising and general central nervous system depressant activity. Flupentixol has been marketed as the dihydrochloride acid salt of the mixed (E)(Z) isomers in tablet form and in solution (Fluanxol®) and also as the (Z) isomer decanoate ester in injectable depot formulations (Depixol®). Flupentixol is a dopamine receptor D₁ and D₂ antagonist and a serotonin receptor 2 antagonist. Flupentixol is used in the treatment of schizophrenia, but also in the treatment of depression, anxiety and severe tension.

Flupentixol is also marketed in a combo tablet together with the antidepressant melitracen under the tradename deanxit®. Melitracen is a tricyclic antidepressant with the structure depicted below 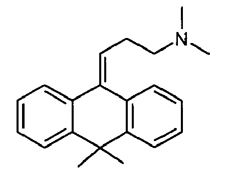 and the systematic name {3-[10,10-dimethyl-10H-anthracen-9-ylidene]-propyl}-dimethylamine. Melitracen was first disclosed in the GB patent 939856 to Kefalas in 1963, and the compound is said to inhibit reuptake of noradrenaline and also to some extent reuptake of serotonin. Deanxit is used e.g. in the treatment of anxiety, depression and asthenia.

Both deanxit® and fluanxol® are sold as sugar coated tablets with a core comprising the active ingredient(s) and lactose monohydrate (15-20%), potato starch (35-40%), gelatine (1-2%), talc (3-5%) and magnesium stearate (0.1-0.5%). The coating comprises gelatine, sucrose and colouring agents.

CN101125131 discloses a solid state form comprising flupentixol and melitracen prepared by ball milling the active ingredients together with diluent to a powder in the absence of solvents.

The present invention provides new compositions comprising flupentixol with improved stability.

### Summary of the invention

The present inventor has surprisingly found that incorporation of cyclodextrin into a solid pharmaceutical composition comprising flupentixol gives rise to an increased stability of flupentixol. Additionally, such compositions are bioequivalent to existing compositions comprising flupentixol, i.e. fluanxol® and Deanxit®. Accordingly, in one embodiment, the present invention relates to a solid pharmaceutical composition comprising flupentixol and pharmaceutically acceptable salts thereof and cyclodextrin.

### Figures

Figure 1: Residual flupentixol in % of amount at t=0 after storage at 40°C and 75% RH for 3 months in open containers. Compositions comprising 0.5 mg flupentixol free base.
Figure 2: Residual flupentixol in % of amount at t=0 after storage at 40°C and 75% RH for 3 months in open containers. Compositions comprising 5 mg flupentixol free base.
Figure 3: Residual flupentixol (absolute amount) after storage at 40°C and 75% RH for 3 months in open containers. Compositions comprising 0.5 mg flupentixol free base and 10 mg melitracen free base.

### Detailed description of the invention

Cyclodextrins are cyclic (α-1,4)-Hnked oligosaccharides of α-D-glucopyranose units derived from starch. The cyclodextrin structure resembles a truncated torus with a central cavity, the size of which depends on the number of glucose units. This structure gives rise to a hydrophilic outer surface and a hydrophobic central cavity. Cyclodextrins are named according to the number of glucose units, so that α-, β- and γ-cyclodextrins have 6, 7 and 8 glucose units, respectively. Numerous cyclodextrin derivatives are also know examples of which include dimethyl-β-cyclodextrin, 2-hydroxyethyl-β-cyclodextrin and 2-hydroxypropyl-β-cyclodextrin [Handbook of Pharmaceutical Excipients (ed. Rowe et al), 5th ed., Pharmaceutical Press]. The different cyclodextrins, i.e. α-, β- and γ-cyclodextrins and the cyclodextrin derivatives are in the present context collectively referred to as "cyclodextrins".

In recent years, cyclodextrins have been used in formulating liquid and solid state drugs e.g. to increase drug solubility and drug stability, and as a taste masker. The underlying mechanism is believed to be the formation of an inclusion complex wherein the drug is trapped in the central cavity [J Pharm. Sci., 85, 1017-1025, 1996*;* J Pharm. Sci., 96, 1691-1707,2007].

Some active pharmaceutical ingredients (API) may be capable of forming such inclusion complex in a dry mixture of cyclodextrin and the API. Others, on the other hand, require that a solution of cyclodextrin and API is prepared for the complex to fully form. While an inclusion complex often seems to confer increased stability to an API, it has also been observed that if an API does not totally fit into the cavity, or is only partially included, a decreased stability is, in fact, observed ["Cyclodextrins and Their Complexes, Chemistry, Analytical Methods, Applications", H Dodziuk (Ed), page 393-396, Wiley-VCH, Weinheim, 2006].

A different approach to improve stability of an API is to decrease the solubility of the API in the tablet environment. It is a common observation that chemical compounds in general, and API's in particular, are more susceptible to chemical reactions, i.e. are less stable in solution than as a solid. Flupentixol has a higher solubility as a salt (i.e. at low pH) than as the free base (i.e. at increased pH). An alternative approach to increase the stability of flupentixol tablets is thus to increase pH in the tablets, e.g. by applying basic excipients, such as bicarbonate.

The manufacture of tablets in large scale normally requires the use of lubricants, the effect of which is to prevent the powder from sticking to the process equipment in general and in particular the tablets from sticking to the punches. Well-known lubricants include talc, silica, magnesium stearate, stearic acid and hydrogenated vegetable oil, such as hydrogenated cotton seed oil. Lubricants are not normally expected to impact the stability of an API.

The data presented in Example 1 shows that in spite of the fact that no pre-formation of a cyclodextrin flupentixol inclusion complex takes place, the use of cyclodextrin in solid flupentixol compositions gives rise to an improved stability. Importantly, said improvement is markedly larger than the stability improvement obtained using other potential stability improving techniques, e.g. pH increase. A pair wise comparison for both high- and low-strengths compositions (M, KM; S, KS; BM, KBM; and BS, KBS) shows that the addition of β-cyclodextrin in 7 out of the 8 comparisons gives rise to an increased stability of flupentixol. It is also noted that the further addition of sterotex, i.e. hydrogenated vegetable oil, rather than magnesium stearate, generally improves the stability of flupentixol. This can be seen from the fact that the KS compositions for both 0.5 mg and 5 mg strengths are the most stable. This is particularly the case for the high-strength composition.

The data presented in example 5 shows that compositions of the present invention comprising flupentixol or flupentixol and melitracen have a superior stability compared to the marketed drugs comprising flupentixol or flupentixol and melitracen, i.e. fluanxol® and deanxit®.

Hence, in one embodiment, the invention relates to a solid pharmaceutical composition comprising flupentixol or a pharmaceutically acceptable salt thereof and cyclodextrin, such as e.g. β-cyclodextrin. In a further embodiment, said composition also comprises hydrogenated vegetable oil, such as hydrogenated cotton seed oil, e.g. sterotex. Examples of flupentixol and pharmaceutically acceptable salts thereof include the hydrochloric acid salt and the di-hydrochloric acid salt. Flupentixol and pharmaceutically acceptable salts thereof, such as e.g. the hydrochloric acid or di-hydrochloric acid may be present in said composition in an amount corresponding to 0.1 - 20 mg free base, such as 0.5 - 10 mg, such as 0.5, 1, 3 or 5 mg.

In addition to cyclodextrin and optionally hydrogenated vegetable oil, a composition of the present invention may also comprise other excipients used in the preparation of solid pharmaceutical preparations. Such excipients include binders, such as starch, sugar, cellulose, microcrystalline cellulose, hydroxypropyl cellulose, lactose, xylitol, sorbitol and mannitol. It also includes disintegrants, such as cross-linked PVP (polyvinyl pyrrolidone), sodium starch glycolate and cross-linked CMC (carboxymethyl cellulose), e.g. croscarmellose sodium. It further includes fillers, such as cellulose, calcium phosphate, lactose, sucrose, glucose, mannitol, sorbitol and calcium carbonate. It further includes glidants, such as silicon dioxide, talcs, and magnesium carbonate. It further includes lubricants, such as talc, silica, hydrogenated vegetable oil and magnesium stearate.

The composition of the present invention may be presented e.g. as tablets, capsules or powders, and particular mention is made of tablets. Such tablets may additionally be coated, e.g. film coated with standard coating materials, such as hydroxyl propylmethyl cellulose, shellac, (partially) hydrolysed PVA (polyvinyl alcohol) and PEG (polyethylene glycol).

In one embodiment, a composition of the present invention comprises β-cyclodextrin, (5-20%), lactose monohydrate (10-25%), starch (45-60%), hydropropyl cellulose (1-10%), microcrystalline cellulose (5-12%), and hydrogenated vegetable oil (0.5-5%) in addition to flupentixol and pharmaceutically acceptable salts thereof equivalent to 0.1-20 mg free base. Optionally, said composition may also comprise cross carmellose sodium (1-10%), talc (0.1-2%), and magnesium stearate (0.1-2%).

In one embodiment, a composition of the present invention comprises β-cyclodextrin, (11-13%), lactose monohydrate (16-18%), starch (48-52%), hydropropyl cellulose (3-5%), microcrystalline cellulose (7-10%) and hydrogenated vegetable oil (0.5-4%) in addition to flupentixol 2HCl corresponding to 0.5, 1, 3 or 5 mg free base. Optionally, said composition also comprises cross carmellose sodium (1-5%), talc (0.3-0.6%), and magnesium stearate (0.1-0.8%).

In one embodiment, a composition of the present invention comprises β-cyclodextrin (5-20%), lactose monohydrate (10-25%), starch (35-45%), hydropropyl cellulose (1-10%), microcrystalline cellulose (5-12%) and hydrogenated vegetable oil (0.5-5%) in addition to flupentixol and pharmaceutically acceptable salts thereof equivalent to 0.1-10 mg free base and melitracen and pharmaceutically acceptable salts thereof equivalent to 1-30 mg free base. Optionally, said composition also comprises cross carmellose sodium (1-10%), talc (0.1-2%), and magnesium stearate (0.1-2%).

In one embodiment, a composition of the present invention comprises β-cyclodextrin, (11-13%), lactose monohydrate (17-20%), starch (38-43%), hydropropyl cellulose (3-5%), microcrystalline cellulose (8-12%) and hydrogenated vegetable oil (0.5-2%) in addition to flupentixol 2 HCl equivalent to 0.5 mg free base and melitracen HCl equivalent to 10 mg free base. Optionally, said composition also comprises cross carmellose sodium (1-3%), talc (0.1-0.8%), and magnesium stearate (0.5-1%).

In one embodiment, a composition of the present invention consists essentially of β-cyclodextrin (5-20%), lactose monohydrate (10-25%), starch (45-60%), hydropropyl cellulose (1-10%), microcrystalline cellulose (5-12%), hydrogenated vegetable oil (0.5-5%), cross carmellose sodium (1-10%), talc (0.1-2%), and magnesium stearate (0.1-2%) in addition to flupentixol and pharmaceutically acceptable salts thereof equivalent to 0.1-20 mg free base.

In one embodiment, a composition of the present invention consists essentially of β-cyclodextrin (11-13%), lactose monohydrate (16-18%), starch (48-52%), hydropropyl cellulose (3-5%), microcrystalline cellulose (7-10%), hydrogenated vegetable oil (0.5-4%), cross carmellose sodium (1-5%), talc (0.3-0.6%), and magnesium stearate (0.1-0.8%) in addition to flupentixol 2HCl corresponding to 0.5, 1, 3 or 5 mg free base.

In one embodiment, a composition of the present invention consists essentially of β-cyclodextrin (5-20%), lactose monohydrate (10-25%), starch (35-45%), hydropropyl cellulose (1-10%), microcrystalline cellulose (5-12%), hydrogenated vegetable oil (0.5-5%), cross carmellose sodium (1-10%), talc (0.1-2%) and magnesium stearate (0.1-2%) in addition to flupentixol and pharmaceutically acceptable salts thereof equivalent to 0.1-10 mg free base and melitracen and pharmaceutically acceptable salts thereof equivalent to 1-30 mg free base.

In one embodiment, a composition of the present invention consists essentially of β-cyclodextrin (11-13%), lactose monohydrate (17-20%), starch (38-43%), hydropropyl cellulose (3-5%), microcrystalline cellulose (8-12%), hydrogenated vegetable oil (0.5-2%), cross carmellose sodium (1-3%), talc (0.1-0.8%) and magnesium stearate (0.5-1%) in addition to flupentixol 2 HCl equivalent to 0.5 mg free base and melitracen HCl equivalent to 10 mg free base.

As mentioned above, flupentixol is already on the market as a single-component tablet (trade name fluanxol®) and as a combo tablet together with melitracen (trade name deanxit®). When a new composition of an existing product is developed, it is a huge advantage if the new and the old composition exhibit the same blood plasma profile upon administration. A therapeutic effect of a compound is closely related to the concentration of said compound in the blood. Thus, if two compositions of the same therapeutically active compound give rise to the same blood concentrations of said compound over time, identical clinical effects from the two compositions can be expected. When two compositions of the same therapeutically active compound give rise to the same blood concentrations of said compound over time, the compositions are said to be bioequivalent. Regulatory authorities allow a simplified access to the market for a new composition of an exiting drug provided the new composition is bioequivalent to an exiting composition. If this is not the case, many regulatory authorities require additional safety and efficacy tests to be conducted before the new composition is allowed on the market, which tests are both expensive and time consuming. In the result, the provision of new compositions of flupentixol and flupentixol/melitracen which compositions are bioequivalent with existing compositions ensure that the clinical effect of the existing compositions are maintained, and it allows a much faster and much cheaper development phase.

The data presented in Example 2 and 3 shows that compositions of the present invention are bioequivalent with exiting compositions of flupentixol and flupentixol and melitracen, i.e. fluanxol® and deanxit®.

As mentioned above, flupentixol is also provided as a combo tablet, i.e. as a tablet which also comprises the tricyclic antidepressant melitracen. The data in example 4 shows the effect of different manufacturing processes for said combo tablet on the stability of flupentixol. In summary, the experiments reported in example 4 assess the effect of mixing melitracen and flupentixol in a wet-granulation step prior to tableting, and mixing two separate granulates, one comprising flupentixol and one comprising melitracen prior to tableting. The data clearly shows that the mixing of two separate granulates (or powders) provides a superior stability. This can be seen from the fact that the stability for KBT is superior to KBV, and that the stability of KT is markedly better than any of the other composition tested.

Thus, in one embodiment, the invention relates to a process for the manufacture of a solid pharmaceutical composition comprising flupentixol or a pharmaceutically acceptable salt thereof and melitracen or a pharmaceutically acceptable salt thereof, the process comprising mixing a first granulate or powder comprising flupentixol or a pharmaceutically acceptable salt thereof and cyclodextrin with a second granulate or powder comprising melitracen or a pharmaceutically acceptable salt thereof.

In a subsequent step, said mixture of granulates or powders obtained above may be compressed to form a tablet, which tablet may be coated. In order to prepare said two granulates, exicipients as discussed above may be applied.

In one embodiment, said first or said second granulate comprise cyclodextrin, such as β-cyclodextrin. In a further embodiment, said first or said second granulate in addition to cyclodextrin, such as β-cyclodextrin also comprise hydrogenated vegetable oil, such as vegetable cotton seed oil.

In one embodiment, a first flupentixol 2 HCl comprising granulate is prepared by mixing flupentixol and lactose monohydrate to obtain a premix comprising 8% flupentixol. Said premix is mixed with β-cyclodextrin, maize starch, lactose monohydrate and hydroxypropyl cellulose, and water is added to obtain a first granulate. A second granulate is prepared by mixing melitracen HCL, maize starch, lactose monohydrate and hydroxypropyl cellulose followed by the addition of water to obtain a granulate. Said first and second granulate are mixed and blended with microcrystalline cellulose, croscarmelose sodium, talc, hydroxypropyl cellulose, talc and hydrogenated begetable oil, and tablets are prepared by compression. As a further step, a coating layer may be added to the tablets.

### Example 1 Effect of cyclodextrin, bicarbonate and lubricants on the stability of flupentixol

Eight compositions were prepared in two strengths of flupentixol (0.5 mg and 5 mg free base) and in order to assess the influence of the excipients on stability, the residual amount of flupentixol were determined after 3 months storage at 40 °C, 75% RH in open containers. Table 1 below summarises the compositions. Flupentixol Premix 8 is a mixture of 8% flupentixol 2HCl in lactose used to improve uniformity of content in the low-strength tablets. Kleptose is β-cyclodextrin; Klucel EXF is hydroxypropyl cellulose with a particle size (D₅₀) around 100 µm; Avicel PH 102 is microcrystalline cellulose with a particle size (D₅₀) around 100 µm; Sterotex is hydrogenated cotton seed oil; and bicarbonate is NaHCO₃.

The compositions were manufactured by preparing one granulate for each of the tablets strengths to which granulates the remaining exicipents were added to achieve the desired final granulates.

### Granulate a - fluanxol 0.5 mg

Flupentixol premix 8 %
Lactose monohydrate
Maize starch
Klucel EXF
Water purified*
   * Solvent used for granulation

### Granulate e-b - fluanxol 5 mg

Flupentixol dihydrochloride
Lactose monohydrate
Maize starch
Klucel EXF
Water purified*
   * Solvent used for granulation
The exipients listed under Granulate a-b were blended in a Diosna P25 high shear mixer for 3 minutes. Mixer speed: 325 rpm/Chopper 2800 rpm. Purified water was sprayed onto the powder in an amount of ~ 22 % w/w. Mixing was continued for about 3 minutes at mixer speed 162 rpm and chopper speed of 2800 rpm, followed by granulation for 30 sec. at mixer speed 325 rpm and chopper speed 2800 rpm.
The granulate obtained was dried in a tray drier. The remaining exipients were added with the exception of Sterotex and magnesium stearate to the dried granulate which was sieved through a mesh size 0.7 mm and blended in a Bohle bin blender for 6 minutes at 22 rpm. Sterotex or magnesium stearate was added (sieved through a sieve size 0.8 mm) and the resulting granulates were blended in a Bohle bin blender for 3 minutes.

Tablets were prepared by compressing the granulates at a Korch PH-106 tableting machine. Target weight for 0.5 mg fluanxol was 60 mg; target weight for 5 mg fluanxol was 250 mg. Hardness for both strengths was 17-39 N

Each tablet comprises 0.584 mg flupentixol 2HCl equivalent to 0.5 mg flupentixol base or 5.83 mg flupentixol 2HCl equivalent to 5 mg flupentixol base.

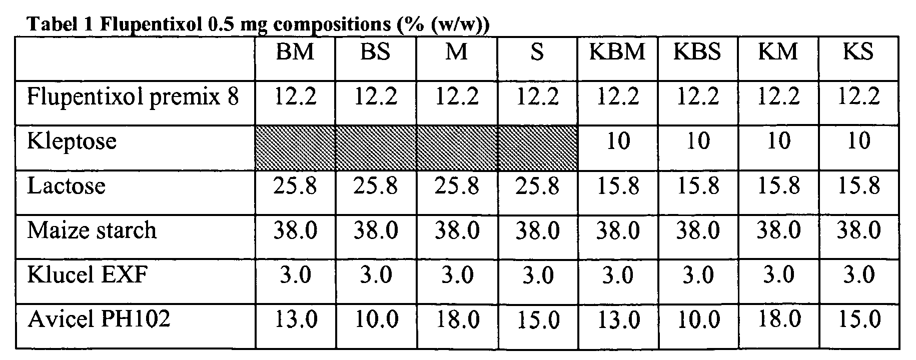

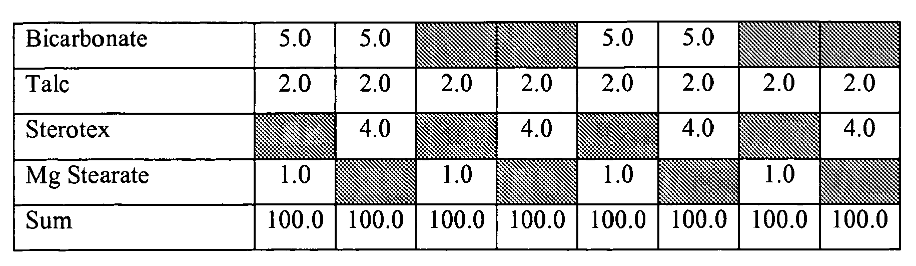

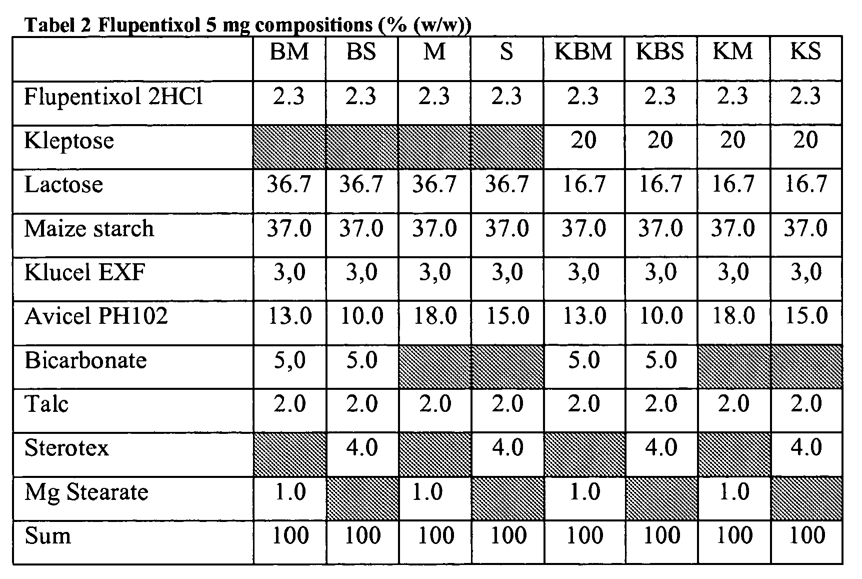

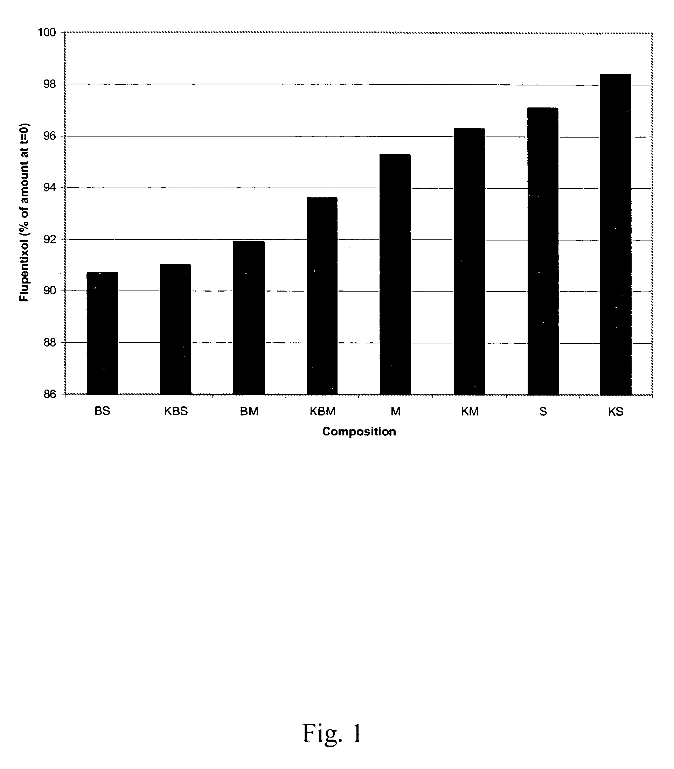

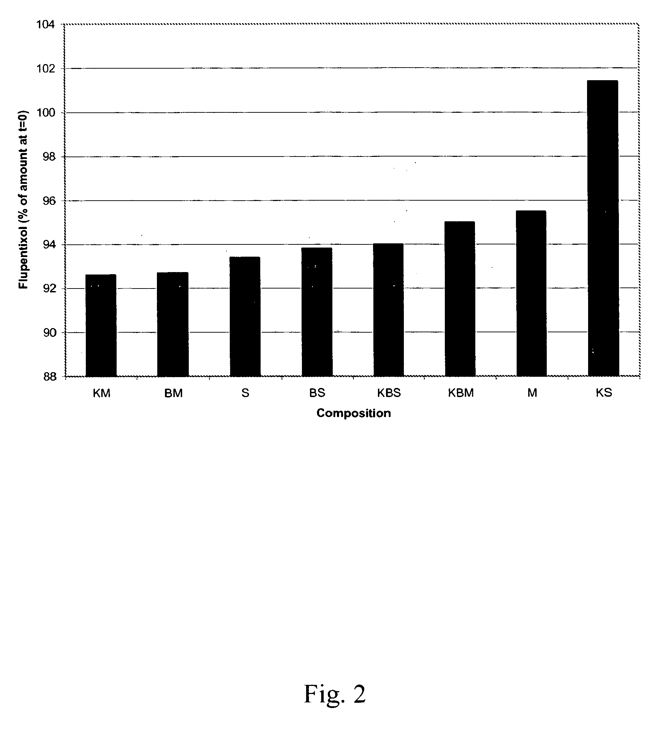

Figure 1 depicts the residual amount in percentage of flupentixol in the eight 0.5 mg flupentixol composition discussed above. Similarly, Figure 2 depicts the residual amount in percentage of flupentixol in the eight 5.0 mg flupentixol compositions discussed above.

The data clearly show that flupentixol compositions comprising β-cyclodextrin are more stable than compositions that do not comprise β-cyclodextrin. The data also show that sterotex has a beneficial impact on the stability of flupentixol.

### Example 2 Bioequivalence of flupentixol composition of present invention and fluanxol®

To investigate whether compositions of the present invention are bioequivalent with fluanxol® a single-dose, open-label, randomised, crossover bioequivalence study in healthy man and women was conducted. 64 patients were enrolled in four cohorts, i.e. 0.5, 1, 3 and 5 mg flupentixol. On the first day, subjects were administered either flupentixol in a composition of the present invention (treatment A) or fluanxol® (treatment B). After a 14 days wash-out period each subject returned to the clinic to receive the alternative treatment (A or B). Blood samples were collected up to 120 hours post-dose in each period and flupentixol plasma concentrations were determined. Bioequivalence was assessed on the basis of the area under the plasma concentration-time curve from zero to infinity (AUC_{0-inf}) and the maximum observed concentration (Cₘₐₓ). One patient withdrew from the 5 mg cohort. The table 3 below summarises the tablet compositions used in treatment A

**Tabel 3**

| | Flupentixol 2 HCl per unit (as free base) | | | |
|---|---|---|---|---|
| | 0.5 mg | 1 mg | 3 mg | 5 mg |
| β cyclodextrin | 14.4 mg | 14.4 mg | 21.6 mg | 36.0 mg |
| Lactose monohydrate | 20.0 mg | 19.85 mg | 30.6 mg | 51.0 mg |
| Maize starch | 62.02 mg | 61.58 mg | 89.7 mg | 49.55 mg |
| Hydroxypropylcellulose | 5.4 mg | 5.4 mg | 6.3 mg | 10.5 mg |
| Microcrystalline cellulose | 12.68 mg | 12.68mg | 14.35 mg | 23.9 mg |
| Croscarmellose sodium | 2.4 mg | 2.4 mg | 7.2 mg | 12.0 mg |
| Talc | 0.6 mg | 0.6 mg | 0.9 mg | 1.5 mg |
| Hydrogenated vegetable oil | 1.2 mg | 1.2 mg | 5.4 mg | 9.0 mg |
| Mg stearate | 0.72 mg | 0.72 mg | 0.36 mg | 0.6 mg |
| Weight of tablet core | 120 mg | 120 mg | 180 mg | 300 mg |

The tablets were coated with 4.4 mg (0.5 mg and 1 mg), 6.6 mg (3 mg) and 9.8 mg(5 mg) coating material consisting of PEG3350, PEG6000, hydrogenated PVA and dye stuff.

The data in Table 4 below shows the calculated AUC_{0-inf} and Cₘₐₓ for the four cohorts.

**Tabel 4**

| | AUC_{0-inf} (h ng/ml) | | Cₘₐₓ (mg/ml) | |
|---|---|---|---|---|
| | Treatment A | Treatment B | Treatment A | Treatment B |
| 0.5 mg | 5.99 | 6.35 | 0.0808 | 0.0856 |
| 1 mg | 13.1 | 13.1 | 0.179 | 0.175 |
| 3 mg | 37.6 | 35.3 | 0.677 | 0.631 |
| 5 mg | 59.5 | 53.3 | 1.41 | 1.23 |

The data presented in table 4 clearly shows that compositions of the present invention are bioequivalent with fluanxol® over a broad range of flupentoxil concentrations.

### Example 3 Bioequivalence of flupentixol/melitracen compositions of present invention and deanxit®

To investigate whether compositions of the present invention are bioequivalent with deanxit® a single-dose, open-label, randomised, crossover bioequivalence study in healthy man and women was conducted. 30 subjects were enrolled. On the first day, subjects were administered either flupentixol and melitracen in a composition of the present invention (treatment A) or deanxit® (treatment B). After a 14 days wash-out period each subject returned to the clinic to receive the alternative treatment (A or B). Blood samples were collected up to 120 hours post-dose in each period and flupentixol and melitracen plasma concentrations were determined. Bioequivalence was assessed on the basis of the area under the plasma concentration-time curve from zero to infinity (AUC_{0-inf}) and the maximum observed concentration (Cₘₐₓ).

Table 5 below summarises the composition of the tablet composition used in treatment A. Each tablet comprised 10 mg melitracen (HCl) and 0.5 mg flupentixol (2HCl), calculated as the free base.

**Tabel 5**

| | |
|---|---|
| β-cyclodextrin | 14.4 mg |
| Lactose monohydrate | 22.056 mg |
| Maize starch | 48.71 mg |
| Hydroxypropylcellulose | 5.4 mg |
| Microcrystalline cellulose | 12.44 mg |
| Croscarmelose sodium | 2.4 mg |
| Talc | 0.60 mg |
| Hydrogenated vegetable oil | 1.2 mg |
| Mg sterate | 0.96 mg |
| Weight of tablet core | 120 mg |

Each tablet was coated with 4.4 mg coating comprising PEG3500, PEG6000, hydrogenated PVA and dye stuff.
Table 6 below shows the AUC_{0-inf} and the Cₘₐₓ for flupentixol and melitracen

**Tabel 6**

| | Flupentixol | | Melitracen | |
|---|---|---|---|---|
| | Treatment A | Treatment B | Treatment A | Treatment B |
| AUC_{0-inf} (h ng/ml) | 8.58 | 8.06 | 292 | 269 |
| Cₘₐₓ Ng/ml) | 0.110 | 0.103 | 8.49 | 8.11 |

The data presented in table 6 clearly shows that compositions of the present invention comprising flupentixol and melitracen are bioequivalent with deanxit® with respect to both flupentixol and melitracen.

### Example 4. Effect of manufacturing process for tablets comprising flupentixol and melitracen on the stability of flupentixol.

Six compositions were prepared as summarised in Table 7 below.

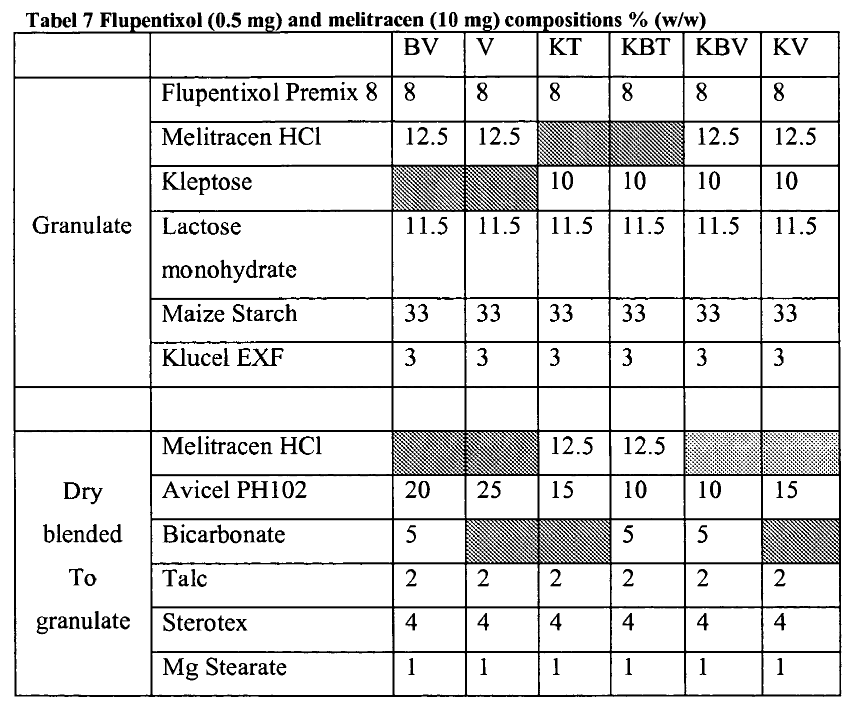

The compositions were obtained by manufacture of three granulates (I,II&III) :

### Granulate I (BV and V)

Flupentixol premix 8 %
Melitracen HCl
Lactose monohydrate
Maize starch
Klucel EXF
Water purified*
   * Solvent used for granulation

### Granulate II (KT and KBT)

Flupentixol premix 8 %
Kleptose
Lactose monohydrate
Maize starch
Klucel EXF
Water purified*
   * Solvent used for granulation

### Granulate III (KBV and KV)

Flupentixol dihydrochloride
Melitracen 2HCl
Kleptose
Lactose monohydrate
Maize starch
Klucel EXF
Water purified*
   * Solvent used for granulation
The exipients were blended in a Diosna P25 high shear mixer for 3 minutes. Mixer speed: 325 rpm/Chopper 2800 rpm. Purified water was sprayed onto the powder in amount of about 22 % w/w. Mixing was continued for 3 minutes at a mixer speed 162 rpm and a chopper speed of 2800 rpm, followed by granulation for 30 sec. at a mixer speed 325 rpm. and a chopper speed 2800 rpm. The granulates obtained were dried in a tray drier. The remaining exipients were added to the fried granulates with the exception of Sterotex and magnesium stearate and sieved through a mesh size 0.7 mm followed by blending in a Bohle bin blender for 6 minutes at 22 rpm. Sterotex and magnesium stearate (sieved through a sieve size 0.8 mm) were added followed by blending in a Bohle bin blender for 3 minutes.

Tablets were compressed at a Korsch PH-106 tabletting machine with a target weight of 90 mg . Hardness: Within 15 - 28 N

Each tablet comprises 0.584 mg flupentixol 2HCl equivalent to 0.5 mg flupentixol base and 11.25 mg melitracen HCl equivalent to 10 mg melitracen base.

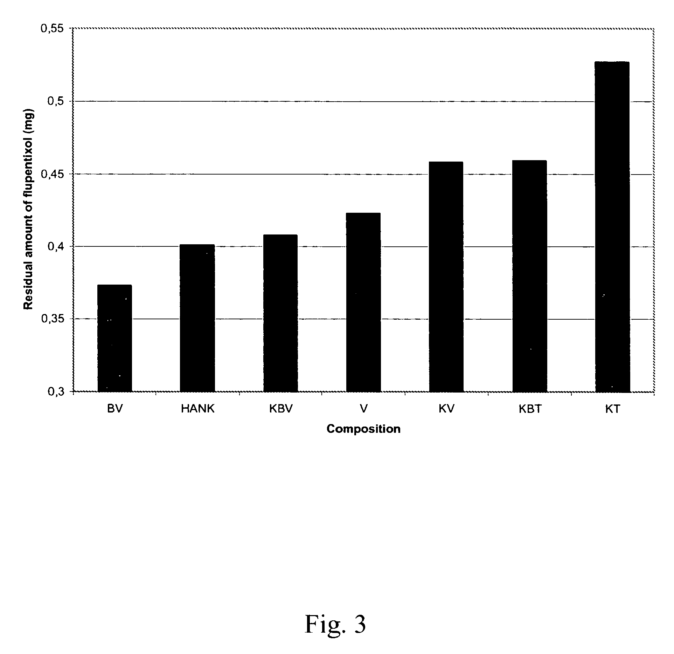

The tablets were placed at 40 °C and 75% RH for three months in open containers following which the residual amount of flupentixol was determined. The data are depicted in Figure 3, which shows the absolute amount of flupentixol in the tablets after the above test period. It is clear, that separate granulation of flupentixol and melitracen prior to tabletting gives rise to superior flupentixol stability. This can be seen from the fact that the compositions KBT and KT, both of which are manufactured using two separate granulates have the best stability. The fact that composition KT, i.e. a composition comprising β-cyclodextrin and sterotex has a markedly better stability than the other compositions lends further support to the conclusions reached from the data presented in Example 1.

### Example 5 Stability of compositions of the present invention and fluanxol® and deanxit®.

Tablets were prepared as in examples 2 and 3 comprising flupentixol 2 HCl corresponding to 1 mg free base/tablet or flupentixol 2HCl corresponding to 0.5 mg free base/tablet and melitracen HCL corresponding to 10 mg free base/tablet. These tablets were subjected to stability testing at 40 °C, 75% RH for three months. The corresponding commercial drugs fluanxol® and deanxit® were also included in the test. The data in Table 8 shows the amount of flupentixol as percentage of the theoretical amount at t=0 and t=3 months.

**Tabel 8**

| | | t=0 month | t=3 months |
|---|---|---|---|
| | | (% of theoretical content) | (% of theoretical content) |
| Flupentixol | Fluanxol® | 102 | 97 |
| | Fluanxol® | 100 | 95 |
| | Fluanxol® | 100 | 95 |
| | Present invention | 99 | 99 |
| | Present invention | 99 | 99 |
| Flupentixol/melitracen | Deanxit® | 101 | 93 |
| | Deanxit® | 100 | 93 |
| | Deanxit® | 101 | 92 |
| | Present invention | 99 | 98 |
| | Present invention | 99 | 97 |

The data presented in table 8 clearly shows that compositions of the present invention are endowed with a superior stability compared to existing commercial compositions of flupentixol or flupentixol and melitracen.

## Claims

1. A solid pharmaceutical composition comprising flupentixol or pharmaceutically acceptable salts thereof and cyclodextrin.

2. The composition according to claim 1 wherein said cyclodextrin is β-cyclodextrin.

3. The composition according to claim 1 or 2, which further comprises hydrogenated vegetable oil.

4. The composition according to any of claims 1-3 which further comprises melitracen or pharmaceutically acceptable salts thereof.

5. The composition according to claim 1, which composition comprises
| | |
|---|---|
| β-cyclodextrin | 5-20% (w/w) |
| Lactose monohydrate | 10-25% (w/w) |
| Starch | 45-60% (w/w) |
| Hydroxypropyl cellulose | 1-10% (w/w) |
| Microcrystalline cellulose | 5-12% (w/w) |
| Hydrogenated vegetable oil | 0.5-5% (w/w) |
in addition to flupentixol or a pharmaceutically acceptable salt thereof corresponding to 0.1-20 mg free base.

6. The composition according to claim 5 which comprises flupentixol 2HCl corresponding to 0.5, 1, 3 or 5 mg free base.

7. The composition according to claim 1, which composition comprises
| | |
|---|---|
| β-cyclodextrin | 5-20% (w/w) |
| Lactose monohydrate | 10-25% (w/w) |
| Starch | 35-45% (w/w) |
| Hydroxypropyl cellulose | 1-10% (w/w) |
| Microcrystalline cellulose | 5-12% (w/w) |
| Hydrogenated vegetable oil | 0.5-5% (w/w) |
in addition to flupentixol or a pharmaceutically acceptable salt thereof corresponding to 0.1-20 mg free base and melitracen or a pharmaceutically acceptable salt thereof corresponding to 1-30 mg free base.

8. The composition according to claim 7 which comprises flupentixol 2HCl corresponding to 0.5 mg free base and melitracen HCl corresponding to 10 mg free base.

9. Use of a composition according to any of claims 1-8 in the manufacture of a medicament for the treatment of schiziophrenia, depression, anxiety, severe tension, or asthenia.

10. A process for the manufacture of a pharmaceutical composition comprising flupentixol or pharmaceutically acceptable salts thereof and melitracen or a pharmaceutically acceptable salt thereof, said process comprising the manufacture of a first granulate or powder containing flupentixol or a pharmaceuticaly acceptable salt thereof and cyclodextrin and a second granulate or powder containing melitracen or a pharmaceutically acceptable salt thereof, followed by mixing of said two granulates or powders and compression of a tablet from the resulting mix.

## Patentansprüche

1. Feste pharmazeutische Zusammensetzung, umfassend Flupentixol oder pharmazeutisch annehmbare Salze davon und Cyclodextrin.

2. Zusammensetzung nach Anspruch 1, wobei das Cyclodextrin β-Cyclodextrin ist.

3. Zusammensetzung nach Anspruch 1 oder 2, weiter umfassend hydriertes pflanzliches Öl.

4. Zusammensetzung nach einem der Ansprüche 1 - 3, weiter umfassend Melitracen oder pharmazeutisch annehmbare Salze davon.

5. Zusammensetzung nach Anspruch 1, wobei die Zusammensetzung Folgendes umfasst:
| | |
|---|---|
| β-Cyclodextrin | 5-20 Gew.% |
| Lactosemonohydrat | 10 - 25 Gew.% |
| Stärke | 45 - 60 Gew.% |
| Hydroxypropylcellulose | 1-10 Gew.% |
| Mikrokristalline Cellulose | 5-12 Gew.% |
| Hydriertes pflanzliches Öl | 0,5 - 5 Gew.% |
zusätzlich zu Flupentixol oder einem pharmazeutisch annehmbaren Salz davon, entsprechend 0,1 - 20 mg freier Base.

6. Zusammensetzung nach Anspruch 5, umfassend Flupentixol 2HCl, entsprechend 0,5, 1, 3 oder 5 mg freier Base.

7. Zusammensetzung nach Anspruch 1, wobei die Zusammensetzung Folgendes umfasst:
| | |
|---|---|
| β-Cyclodextrin | 5-20 Gew.% |
| Lactosemonohydrat | 10 - 25 Gew.% |
| Stärke | 35 - 45 Gew.% |
| Hydroxypropylcellulose | 1 - 10 Gew.% |
| Mikrokristalline Cellulose | 5 - 12 Gew.% |
| Hydriertes pflanzliches Öl | 0,5 - 5 Gew.% |
zusätzlich zu Flupentixol oder einem pharmazeutisch annehmbaren Salz davon, entsprechend 0,1 - 20 mg freier Base und Melitracen oder einem pharmazeutisch annehmbaren Salz davon, entsprechend 1 - 30 mg freier Base.

8. Zusammensetzung nach Anspruch 7, umfassend Flupentixol 2HCl, entsprechend 0,5 mg freier Base und Melitracen HCl entsprechend 10 mg freier Base.

9. Verwendung einer Zusammensetzung nach einem der Ansprüche 1 - 8 bei der Herstellung eines Arzneimittels für die Behandlung von Schizophrenie, Depression, Angstzuständen, schwerer Anspannung oder Asthenie.

10. Verfahren zur Herstellung einer pharmazeutischen Zusammensetzung, umfassend Flupentixol oder pharmazeutisch annehmbare Salze davon und Melitracen oder ein pharmazeutisch annehmbares Salz davon, wobei das Verfahren die Herstellung eines ersten Granulats oder Pulvers umfasst, enthaltend Flupentixol oder ein pharmazeutisch annehmbares Salz davon und Cyclodextrin und eines zweiten Granulats oder Pulvers, das Melitracen oder ein pharmazeutisch annehmbares Salz davon enthält, gefolgt von der Mischung der zwei Granulate oder Pulver und Kompression einer Tablette aus der sich ergebenden Mischung.

## Revendications

1. Composition pharmaceutique solide comprenant du flupentixol ou des sels pharmaceutiquement acceptable de celui-ci et de la cyclodextrine.

2. Composition selon la revendication 1, dans laquelle ladite cyclodextrine est la β-cyclodextrine.

3. Composition selon la revendication 1 ou 2, qui comprend en outre une huile végétale hydrogénée.

4. Composition selon l'une quelconque des revendications 1 à 3, qui comprend en outre du mélitracène ou des sels pharmaceutiquement acceptables de celui-ci.

5. Composition selon la revendication 1, laquelle composition comprend
| | |
|---|---|
| β-cyclodextrine | 5 à 20% (p/p) |
| Lactose monohydraté | 10 à 25% (p/p) |
| Amidon | 45 à 60% (p/p) |
| Hydroxypropylcellulose | 1 à 10% (p/p) |
| Cellulose microcristalline | 5 à 12% (p/p) |
| Huile végétale hydrogénée | 0,5 à 5% (p/p) |
en plus du flupentixol ou d'un sel pharmaceutiquement acceptable de celui-ci correspondant à 0,1 à 20 mg de base libre.

6. Composition selon la revendication 5 qui comprend du flupentixol 2 HCl correspondant à 0,5, 1, 3 ou 5 mg de base libre.

7. Composition selon la revendication 1, laquelle composition comprend
| | |
|---|---|
| β-cyclodextrine | 5 à 20% (p/p) |
| Lactose monohydraté | 10 à 25% (p/p) |
| Amidon | 35 à 45% (p/p) |
| Hydroxypropylcellulose | 1 à 10% (p/p) |
| Cellulose microcristalline | 5 à 12% (p/p) |
| Huile végétale hydrogénée | 0,5 à 5% (p/p) |
en plus du flupentixol ou d'un sel pharmaceutiquement acceptable de celui-ci correspondant à 0,1 à 20 mg de base libre et du mélitracène ou d'un sel pharmaceutiquement acceptable de celui-ci correspondant à 1 à 30 mg de base libre.

8. Composition selon la revendication 7 qui comprend du flupentixol 2 HCl correspondant à 0,5 mg de base libre et du mélitracène HCl correspondant à 10 mg de base libre.

9. Utilisation d'une composition selon l'une quelconque des revendications 1 à 8 dans la fabrication d'un médicament destiné au traitement de la schizophrénie, de la dépression, de l'anxiété, d'une tension sévère ou d'une asthénie.

10. Procédé de fabrication d'une composition pharmaceutique comprenant du flipentixol ou des sels pharmaceutiquement acceptables de celui-ci et du mélitracène ou un sel pharmaceutiquement acceptable de celui-ci, ledit procédé comprenant la fabrication d'un premier granulat ou d'une première poudre contenant le flupentixol ou un sel pharmaceutiquement acceptable de celui-ci et la cyclodextrine et d'un second granulat ou d'une seconde poudre contenant le mélitracène ou un sel pharmaceutiquement acceptable de celui-ci, ceci suivi du mélange desdits deux granulats ou desdites deux poudres et de la compression d'un comprimé à partir du mélange résultant.
